(19) Europäisches Patentamt
European Patent Office
Office européen des brevets

(11) EP 0 918 046 B2

(12) NEW EUROPEAN PATENT SPECIFICATION

(45) Date of publication and mention of the opposition decision:
**14.03.2007 Bulletin 2007/11**

(45) Mention of the grant of the patent:
**03.04.2002 Bulletin 2002/14**

(21) Application number: **98309209.9**

(22) Date of filing: **11.11.1998**

(51) Int Cl.:
***C07C 39/16*** *(2006.01)* ***C07C 37/20*** *(2006.01)*

# (54) Method for manufacturing bisphenol

Verfahren zur Herstellung von Bisphenol-A

Procédé de préparation de bisphénol-A

(84) Designated Contracting States:
**BE DE ES FR GB IT NL**

(30) Priority: **19.11.1997 JP 31812597**

(43) Date of publication of application:
**26.05.1999 Bulletin 1999/21**

(73) Proprietor: **GENERAL ELECTRIC COMPANY**
**Schenectady, NY 12345 (US)**

(72) Inventors:
- **Kono, Kiyoshi**
  **Ichihara City,**
  **Chiba Pref. 299-0125 (JP)**
- **Uno, Kazutoyo**
  **Chiba City,**
  **Chiba Pref. 263-0024 (JP)**
- **Minami, Satoru**
  **Ichihara City,**
  **299-0125 Chiba Prefecture (JP)**
- **Shimoda, Tomoaki**
  **Ichihara City,**
  **Chiba Pref. 299-0125 (JP)**

(74) Representative: **Szary, Anne Catherine**
**London Patent Operation**
**General Electric International, Inc.**
**15 John Adam Street**
**London WC2N 6LU (GB)**

(56) References cited:
**EP-A- 0 719 814** **GB-A- 1 384 840**

Remarks:
The file contains technical information submitted after the application was filed and not included in this specification

EP 0 918 046 B2

## Description

The present invention concerns a method for manufacturing a bisphenol in which there is no residue of the organic catalysts ordinarily used in manufacturing bisphenols, so that byproducts are not produced during purification, allowing bisphenols with outstanding color tone, thermal resistance, etc., to be obtained.

Bisphenols such as bisphenol A and bisphenol F have conventionally been widely used as raw materials for manufacturing polymers such as polycarbonate, epoxy resins, and polyarylate.

These bisphenols, such as bisphenol A, are ordinarily manufactured by reacting phenol and acetone in the presence of a mineral acid such as hydrochloric acid or an acidic catalyst such as a strongly-acidic ion-exchange resin. The various similar methods for making bisphenol F which is based on fluorenone, an aromatic ketone, are also well described in the patent literature.

In manufacturing bisphenols using a strongly acidic ion-exchange resin as a catalyst, there were cases in which minute amounts of acidic substances were eluted from the strongly acidic ion-exchange resin catalyst. Moreover, in manufacturing of bisphenols using mineral acid catalysts, the acidic substances could be removed from the bisphenol obtained, but it was difficult to completely remove these acidic substances.

The amount of acidic substances in bisphenols obtained in this manner is taken to be 2 ppm or less, on a p-toluene sulfonate basis by acid titration, in the bisphenol ordinarily manufactured at commercial plants, but even with this amount, the bisphenols were discolored, and discoloration of polymers in which these bisphenols were used also occurred. This is considered attributable to factors such as thermal decomposition of bisphenols in the bisphenol purification process due to residual acidic substances in the bisphenol.

Furthermore, when minute amounts of acidic substances are present in the bisphenol and such bisphenol is used as a raw material for manufacturing polymers such as polycarbonate by transesterification, this gives rise to problems such as major changes in the degree of polymerization and impairment of the physical properties of the polymer obtained due to these acidic substances.

In order to solve these problems, the inventors of the present invention proposed a method in Japanese Unexamined Patent Application H8-183844 in which, in manufacturing of polycarbonate by melt polycondensation, (1) an addition product of purified bisphenol and phenol is formed, (2) an alkali metal compound and/or alkaline earth metal compound is added to the additional compound obtained as a catalyst in the amount of $5 \times 10^{-8}$ to $2 \times 10^{-6}$ moles with respect to 1 mole of the bisphenol and then dispersed or dissolved, (3) the phenol is removed from said addition compound, and (4) polycarbonate is manufactured using the bisphenol obtained.

However, in the method for purifying bisphenol presented in the above-mentioned patent application, the alkali metal compound and/or alkaline earth metal compound was added in an amount within the above specified range with respect to 1 mole of bisphenol. It was discovered by the inventors that in bisphenol purified in this manner, the bisphenol undergoes thermal decomposition, etc., during the purification process, and when polymers such as polycarbonate are manufactured using bisphenols obtained in this manner, variations occur in the color tone and thermal resistance of the polymer obtained.

The inventors of the present invention conducted studies on this problem, and they found that the amount of acidic substances contained as impurities in crude bisphenol varies, and that for this reason, by adding a specified amount of an alkali metal compound and/or alkaline earth metal compound, it is possible to obtain bisphenol having sufficiently outstanding properties. They perfected the present invention based on this finding.

The purpose of the present invention is to provide a method for manufacturing a bisphenol in which there is no residue of the organic catalysts ordinarily used in manufacturing bisphenols, so that byproducts are not produced during purification, allowing bisphenols with outstanding color tone, thermal resistance, etc., to be obtained

The present invention comprises a process for manufacturing a bisphenol by reacting a phenol and a ketone comprising:

> adding to the bisphenol obtained by reacting the phenol and the ketone, an alkali metal compound or alkaline earth metal compound;
> thereby adjusting the basicity to be equivalent to a bisphenol/bisphenol disodium salt mixture comprising an amount of from $1 \times 10^{-8}$ to $1 \times 10^{-6}$ moles of bisphenol disodium salt with respect to 1 mole of bisphenol, wherein the amount of the alkali metal compound or alkaline earth metal compound added is controlled based on measuring the basicity of the bisphenol finally obtained.

The aforementioned basicity of the bisphenol can be determined based on the degree of transesterification in transesterification of the bisphenol and a carbonic diester using the alkali metal compound and/or alkaline earth metal compound contained in said bisphenol as a transesterification catalyst.

A preferred embodiment of the present invention is a method for manufacturing bisphenol comprising the following processes:

(a) a phenol and a ketone are reacted in the presence of an acidic catalyst to form bisphenol,
(b) the catalyst and low-boiling-point substances are removed from the reaction mixture containing the bisphenol formed,
(c) a homogeneous solution of bisphenol with an adjusted concentration is obtained by adding or removing phenol,
(d) the homogeneous solution obtained as described above is cooled and an addition compound of bisphenol and phenol is crystallized to form a slurry,
(e) the slurry is subjected to solid-liquid separation to obtain an addition compound of bisphenol and phenol in the form of a solid,
(f) the solid addition compound is heated and melted, and
(g) the phenol is removed from the molten substance;

characterized in that the alkali metal compound and/or alkaline earth metal compound is added in at least one of the processes from (c) through (f).

The amount of the alkali metal compound and/or alkaline earth metal compound added is controlled based on the basicity of the bisphenol finally obtained.

In the present invention, the bisphenol should preferably be bisphenol A or bisphenol F.

In the present invention, the basicity of the bisphenol should preferably be controlled to within 10% of a specified basicity selected from the range of 1 x $10^{-8}$ to 1 x $10^{-6}$ moles of bisphenol as disodium salt with respect to 1 mole of the bisphenol.

Figure 1 is a schematic process flow diagram of a preferred method for manufacturing bisphenols according to the present invention.

The following is a specific explanation of the method for manufacturing bisphenol according to the present invention.

In the method of the present invention, in manufacturing of bisphenol by reacting phenols and ketones, an alkali metal compound and/or alkaline earth metal compound is added to bisphenol obtained by reacting a phenol and a ketone, and the basicity of the bisphenol is adjusted so as to be equivalent to an amount of 1 x $10^{-8}$ to 1 x $10^{-6}$ moles of bisphenol as disodium salt with respect to 1 mole of bisphenol.

In the present invention, bisphenol is produced by reacting a phenol and a ketone according to processes (a) through (g) below, and after the formation of an addition compound of the bisphenol and phenol obtained, the phenol is then removed to purify the bisphenol. In this process, an alkali metal compound and/or alkaline earth metal compound should preferably be added prior to removing the phenol from the addition compound in order to obtain bisphenol adjusted to the aforementioned basicity. This type of preferred specific embodiment will now be explained with reference to the process flow chart shown in Fig. 1.

Process (a)

In the present invention, phenols and ketones are first reacted to form liquid bisphenols.

In the present invention, for example, the bisphenol obtained by reacting phenols and ketones may have the formula below.

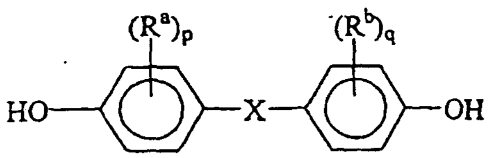

in the formula, $R^a$ and $R^b$ are halogens or monovalent hydrocarbon groups, and they may be the same or different, p and q are integers from 0 to 4,
X is

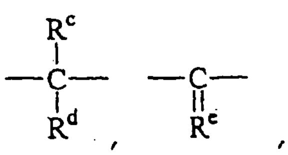

$R^c$ and $R^d$ are hydrogen atoms or monovalent hydrocarbon groups, a ring structure may be formed by $R^e$ and $R^d$, and $R^e$ is a divalent hydrocarbon group.

Specific examples of the bisphenol of the formula above include bis(hydroxyaryl)alkanes such as

1,1-bis(4-hydroxyphenyl)methane,
1,1-bis(4-hydroxyphenyl)ethane,
2,2-bis(4-hydroxyphenyl)propane (referred to in the following as bisphenol A),
2,2-bis(4-hydroxyphenyl)butane,
2,2-bis(4-hydroxyphenyl)octane,
1,1-bis(4-hydroxyphenyl)propane,
1,1-bis(4-hydroxyphenyl)n-butane,
bis(4-hydroxyphenyl)phenylmethane,
2,2-bis(4-hydroxy-1-methylphenyl)propane,
1,1-bis(4-hydroxy-t-butylphenyl)propane,
and 2,2-bis(4-hydroxy-3-bromophenyl)propane,
and bis(hydroxyaryl)cycloalkanes such as
1,1-bis(4-hydroxyphenyl)cyclopentane and
1,1-bis(4-hydroxyphenyl)cyclohexane.

Moreover, in the present invention, bisphenol may be manufactured in such a manner that in the above formula, X is -O-, -S-, -SO-, or $-SO_2-$, with examples of compounds that can be manufactured including bis(hydroxyaryl) ethers such as

4,4'-dihydroxydiphenyl ether and
4,4'-dihydroxy-3,3'-dimethylphenyl ether,
bis(hydroxydiaryl) sulfides such as
4,4'-dihydroxydiphenyl sulfide and
4,4'-dihydroxy-3,3'-dimethyldiphenyl sulfide,
bis(hydroxydiaryl) sulfoxides such as
4,4'-dihydroxydiphenyl sulfoxide and
4,4'-dihydroxy-3,3'-dimethyldiphenyl sulfoxide,
and bis(hydroxydiaryl) sulfones such as
4,4'-dihydroxydiphenyl sulfone and
4,4'-dihydroxy-3,3'-dimethyldiphenyl sulfone.

Among these substances, manufacturing of bisphenol A is particularly preferred.

Bisphenols such as those described above may be obtained by a commonly-known bisphenol synthesis method in which phenols and ketones are condensed in the presence of an acidic catalyst. Phenols having a structure in which there is not a bond with X in the formula above may be used. Moreover, if the above bisphenols can be obtained, one may also carry out condensation of phenols with formaldehyde, sulfonic acids, etc.

The following is an explanation of manufacturing of bisphenol A, mainly by dehydration-condensation of phenol and acetone.

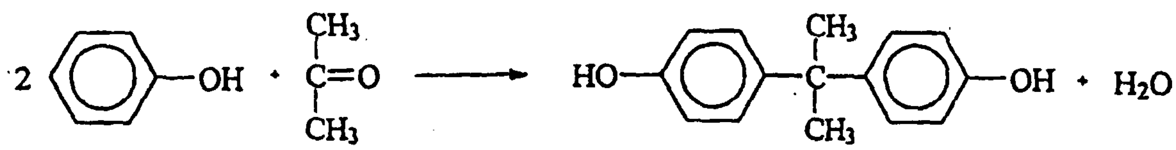

In the above reaction, the phenol is ordinarily used in an excess amount with respect to the acetone, and the molar ratio of the phenol to the acetone (phenol/acetone) should ordinarily be 3/30, and preferably 5/20.

There are no particular restrictions on the acidic ion-exchange resin catalyst used as an acidic catalyst, and any commonly-known catalyst of this type may be used, but ordinarily, one should preferably use a sulfonic acid-type cation-exchange resin having a degree of crosslinking in a gel mold of from about 1 to about 8%, and preferably from about 2 to about 6%.

Moreover, a mineral acid catalyst such as sulfuric acid may also be used.

The reaction of phenol and acetone is ordinarily carried out at a temperature of 30-100°C, and preferably 50-90°C, at a pressure ranging from normal pressure to 490 kPa to (5 $kg/cm^2G$).

In the reaction of the above phenol and acetone, one ordinarily obtains a liquid reaction mixture containing, in addition to bisphenol A, reaction byproducts such as unreacted phenol, unreacted acetone, and water produced as a byproduct.

Process (b)

Catalysts and low-boiling-point substances are removed from the reaction mixture containing liquid bisphenol obtained as described above.

In cases where a mineral acid is used as the above reaction catalyst, prior to removing the low-boiling-point substances from the reaction mixture by distillation, a process for removing the catalyst such as washing with water is carried out. In reactions using a fixed-bed reactor filled with an ion-exchange resin catalyst, as a reaction mixture is obtained which does not contain the catalyst, processing to remove the catalyst is not carried out.

Distillation of the reaction mixture is ordinarily carried out at from 6.65-39.9 kPa (50 to 300 mmHg) and from 70 to 130°C. In this reduced-pressure distillation, together with low-boiling-point substances such as acetone and water, a portion of the phenol is also removed by azeotropy.

Process (c)

A homogeneous bisphenol solution with an adjusted concentration is obtained by the addition or removal of phenols.

Among such phenols, phenol is preferred, and it is preferable, for example, to produce an addition compound of bisphenol A and phenol.

In order to efficiently crystallize the addition compound of bisphenol A and phenol, a homogeneous solution of bisphenol A and phenol should be formed, and the concentration of bisphenol A in this homogeneous solution should be from about 20 to about 50% by weight, and preferably from about 30 to about 45% by weight. This homogeneous solution may be composed of the aforementioned addition compound or of a mixture of this addition compound and phenol.

Process (d)

The homogeneous solution obtained as described above is cooled and an addition compound of bisphenol and phenol is crystallized to form a slurry.

A homogeneous solution of bisphenol and phenol should preferably be cooled to from about 35 to about 60°C, and cooling should preferably be carried out using an external heat exchanger or by heat extraction at reduced pressure.

Process (e)

The slurry obtained as described above is subjected to solid-liquid separation, and an addition compound of bisphenol and phenol is obtained.

Solid-liquid separation of the slurry may be carried out by methods such as centrifugation and vacuum filtration. By means of this solid-liquid separation, the crystals of the addition product of bisphenol and phenol are separated from the mother liquor, which contains reaction byproducts, etc. The separated addition compound (wet cake) may also be washed with phenol, etc.

Process (f)

The solid addition product obtained as described above is heated and melted.

Addition product crystals separated as described above, e.g., an addition product of bisphenol A and phenol, is ordinarily heated and melted at 100-160°C to obtain a mixed solution (molten mixture).

Process (g)

The phenol is removed from the mixed solution (molten mixture) by a process such as vacuum distillation, and the bisphenol is recovered. In removing the phenol by distillation, vacuum distillation is carried out at a pressure of from about 1,33-13,3 kPa (10 to about 100 mmHg) and a distillation temperature of from about 150 to about 190°C. This operation is carried out at a temperature at least about 10°C higher than the melting point of the mixed solution of bisphenol A and phenol in the distillation tower.

In process (g), it is also possible to remove the phenol present in the bisphenol A by steam stripping, etc., using the methods presented in documents such as Japanese Unexamined Patent Applications H2-28126 and S63-132850.

The bisphenol A should preferably be manufactured carrying out the above process continuously.

In the present invention, in manufacturing bisphenol A as described above, in at least one of the processes from process (c) to process (f), an alkali metal compound and/or alkaline earth metal compound (sometimes referred to in the following as an alkali(ne earth) metal compound) is added, and the basicity of the bisphenol is adjusted to correspond to the amount of from about 1 x $10^{-8}$ to about 1 x $10^{-6}$ moles of bisphenol as disodium salt with respect to 1 mole of the bisphenol.

An organic acid salt, inorganic acid salt, oxide, hydroxide, hydride, or alcoholate, etc., of an alkali metal and/or alkaline earth metal may be mentioned as preferred examples of the alkali metal compound and/or alkaline earth metal compound used in the present invention.

Specific examples of alkali metal compounds include sodium hydroxide, potassium hydroxide, lithium hydroxide, sodium hydrogencarbonate, potassium hydrogencarbonate, lithium hydrogencarbonate, sodium carbonate, potassium carbonate, lithium carbonate, sodium acetate, potassium acetate, lithium acetate, sodium stearate, potassium stearate, lithium stearate, sodium boron hydride, lithium boron hydride, sodium boron phenylate, sodium benzoate, potassium benzoate, lithium benzoate, disodium hydrogenphosphate, dipotassium hydrogenphosphate, dilithium hydrogenphosphate, disodium salts, dipotassium salts, and dilithium salts of bisphenol A, and sodium salts, potassium salts, and lithium salts of phenol, etc.,
and specific examples of alkaline earth metal compounds include calcium hydroxide, barium hydroxide, magnesium hydroxide, strontium hydroxide, calcium hydrogencarbonate, barium hydrogencarbonate, magnesium hydrogencarbonate, strontium hydrogencarbonate, calcium carbonate, barium carbonate, magnesium carbonate, strontium carbonate, calcium acetate, barium acetate, magnesium acetate, strontium acetate, and strontium stearate, etc. These compounds may be used in combinations of two or more.

The aforementioned alkali or alkaline earth metal compound should preferably be used in a molten state, e.g., an aqueous solution, a solution of an alcohol such as methanol or ethanol, or a phenol solution of the alkali or alkaline earth metal compound may be used. Moreover, bisphenol containing the aforementioned alkali alkaline earth metal compound may also be used.

By means of adding the aforementioned alkali or alkaline earth metal compound during one of the processes from process (c) to process (f), the basicity of the bisphenol is adjusted, and the alkali or alkaline earth metal compound may be added, for example, to the homogeneous solution obtained in process (c), the slurry obtained in process (d), a wash solution of the addition compound subjected to solid-liquid separation (wet cake) in process (e), or the molten substance (liquid mixture) obtained in process (f).

In this case, the basicity of the bisphenol is ordinarily directly determined by titration, but it is extremely difficult to measure the above basicity corresponding to extremely minute amounts of sodium by titration. The method of determining the basicity of this bisphenol as the amount of the alkali(ne earth) metal compound contained in the bisphenol which is effectively used as a transesterification catalyst is considered to be an accurate and optimal method.

Specifically, this determination may be carried out as follows.

First, a calibration curve is prepared showing the relationship between the amount of the catalyst when the bisphenol and the carbonic diester are subjected to transesterification (amount effectively used as a catalyst) and the degree of transesterification. In this case, the amount of disodium salt of bisphenol with respect to 1 mole of pure bisphenol is taken as the amount of the catalyst. Pure bisphenol refers to bisphenol which contains essentially no acidic or basic impurities. The degree of transesterification may be determined, for example, based on the amounts of phenol, oligomers, unreacted bisphenol, carbonic diesters, etc., produced in the reaction. The amount of the phenol, oligomers, unreacted bisphenol, or diphenylcarbonate may be measured by methods such as using a near infrared meter, measuring the index of diffraction, or high-performance liquid chromatography.

By measuring the degree of transesterification in transesterification of the bisphenol containing an alkali or alkaline earth metal compound manufactured as described above and a carbonic diester under the same conditions as the transesterification reaction used in preparing the calibration curve (temperature, pressure), the basicity of the bisphenol may be determined from the aforementioned calibration curve based on the amount of the alkali or alkaline earth metal compound in the bisphenol effectively used as a transesterification catalyst, i.e., the amount corresponding to the content of disodium salt of bisphenol with respect to 1 mole of purified bisphenol.

This measurement of the basicity of the bisphenol may be carried out at fixed intervals, but one should preferably install the aforementioned analysis unit on a bisphenol manufacturing line and continuously measure the basicity of the manufactured bisphenol.

By measuring the basicity of the bisphenol finally obtained as described above and controlling the amount of the alkali alkaline earth metal compound added in processes (c)-(d) based on this measurement value, the basicity of the bisphenol can be adjusted.

In the present invention, by means of the method described above, the basicity of the bisphenol is adjusted

within the range of $1 \times 10^{-8}$-$1 \times 10^{-6}$ moles of bisphenol as disodium salt per mole of bisphenol, but it should preferably be adjusted to within about 10%, and even more preferably, about 6% of a specified basicity value selected from this range.

Molten bisphenol is obtained in the aforementioned bisphenol manufacturing process (g). This molten bisphenol is then ordinarily subjected to cooling and pelletization in process (h). Pelletization of the bisphenol is carried out by a method such as atomization, dripping, or spraying of the molten bisphenol obtained in process (g) using a spray dryer, etc., to obtain liquid drops, and then cooling and solidifying these drops using nitrogen, air, etc.

The purity of purified bisphenol obtained as described above should be 99% by weight or more, and preferably 99.5% or more, in the case of bisphenol A as measured by high-performance liquid chromatography (HPLC).

In the present invention, moreover, bisphenol having outstanding color tone is obtained.

By means of the present invention, there is no residue of the organic catalysts ordinarily used in manufacturing bisphenol, so that byproducts are not produced during purification, allowing bisphenol with outstanding color tone, thermal resistance, etc., to be obtained.

The bisphenol A obtained in the present invention is well-suited as a raw material for manufacturing of polymers such as polycarbonate, epoxy resin, and polyarylate.

Examples

The following is a specific explanation of the present invention by means of examples, but the invention is not limited to these examples.

In this specification, the basicity, color tone, and purity of the bisphenol A manufactured in the working examples and the MFR and color tone of the polycarbonate manufactured using bisphenol A were measured as follows.

Basicity of Bisphenol A

Bisphenol A (abbreviated in the following as BPA) manufactured in the working examples and diphenol carbonate were continuously fed into a 100 ml scale reactor at a molar ratio of 1 : 1 at a total rate of 200 ml/hr, transesterification was carried out at 160°C with a retention time of 30 minutes, the liquid discharged from the reactor was measured using a near infrared meter, the concentration of phenol in this liquid was measured (amount of phenol produced in the reaction, i.e., degree of transesterification), and the basicity of the bisphenol A was determined using the following calibration curve prepared in advance under the same transesterification conditions.

$$\text{Basicity of bisphenol A (moles of disodium salt of bisphenol A/moles of BPA)} = 3.33 \times 10^{-7} \times \text{phenol concentration (\% by weight)}$$

Color Tone of Bisphenol A

The color tone of the bisphenol A after heating for 10 minutes in air at 250°C was visually measured using APHA standard colorimetric solution.

Purity of Bisphenol A

Purity was measured by high-performance liquid chromatography (HPLC).

Color tone of polycarbonate (yellowing index: YI)

An injection-molded test piece 3 mm in thickness was molded with a cylinder temperature of 290°C, an injection pressure of 98 mPa (1,000 kg/cm$^2$) a cycle time of 45 seconds, and a mold temperature of 100°C, the X, Y, and Z values were measured by the transmission method using the ND-1001 DP Colorand Color Difference Meter manufactured by Nihon Denshoku Kogyo K.K., and yellowing index [YI] was measured.

$$YI = 100 (1.277 X - 1.060 Z)/Y$$

MFR of polycarbonate

This was measured at a temperature of 250°C and a load of 1.2 kg according to the method of JIS K-7210.

In the examples, bisphenol A and polycarbonate were continuously manufactured, essentially by the following process.

Manufacturing process of bisphenol A

(a) Phenol and acetone were reacted at a molar ratio of phenol/acetone equal to about 5 at 50°C and ordinary pressure using a sulfonic acid-type cation-exchange resin with a degree of crosslinking of 4% as an acidic catalyst to obtain a mixed solution.
(b) The reaction solution obtained above was vacuum-distilled at 26.6 kPa (200 mmHg) and 120°C, and the unreacted acetone, byproduct water, etc., were removed to obtain a crude solution of bisphenol A.
(c) The phenol was removed from the crude solution obtained above, and the concentration of bisphenol A was adjusted to 30% by weight.
(d) The crude solution having its concentration adjusted as described above (homogeneous solution) was subjected to heat extraction using an external heat exchanger and cooled to 42°C, and an addition compound of bisphenol A and phenol was crystallized, forming a slurry.
(e) The slurry was separated into an addition compound (crystalline portion) and the mother liquor by centrifugation and vacuum filtration.
(f) After being subjected to solid-liquid separation, the addition compound of bisphenol A and phenol was heated and melted at 130°C.
(g) The molten material (liquid mixture) was vacuum-distilled at 13.3 kPa (100 mmHg) and 190°C to remove the phenol, and molten bisphenol A was recovered.
(h) The molten bisphenol A obtained in process (g) above was made into liquid drops using a spray dryer and cooled and solidified with nitrogen to obtain bisphenol A pellets.

Polycarbonate Manufacturing Process

The device used for polymerization of polycarbonate was equipped with one stirring tank for mixing of the raw materials, two prepolymerization tanks, and two lateral polymerization tanks. The reaction conditions were as follows.

| Reactor | Pressure | Temperature (°C) | Average retention time (hrs) |
|---|---|---|---|
| Stirring tank | Nitrogen atmosphere | 160 | 2.0 |
| Prepolymerization tank I | (100 torr) 13.3 kPa | 230 | 1.0 |
| Prepolymerization tank II | (20 torr) 2.7 kPa | 240 | 0.5 |
| Lateral polymerization tank I | (3~5 torr) 0.4-0.7 kPa | 270 | 0.5 |
| Lateral polymerization tank II | (0.1~1.0 torr) 13.3-133 Pa | 275 | 0.5 |

From the above-mentioned bisphenol A manufacturing device, molten bisphenol A was fed from process (g) via a direct line or pelletized bisphenol A (supply rate 36.0 kg/hr) and molten diphenylcarbonate fed via a direct line after distillation (supply rate 34.7 kg/hr) were continuously supplied to the stirring tank maintained at the above temperature, and at a supply rate of 36.0 kg/hr on a bisphenol A basis, the mixture was successively fed to prepolymerization tank I, prepolymerization tank II, lateral polymerization tank I, and lateral polymerization tank II, and polymerization was carried out under the above conditions to manufacture polycarbonate having a target MFR of 11.0 g/10 min.

While monitoring the measured MFR at 2-hour intervals, the pressure of lateral polymerization tank I and lateral polymerization tank II was monitored so as to operate with as little deviation as possible from the target MFR.

Example 1

<u>Manufacturing of bisphenol A</u>

In process (c) above, a 0.5% sodium hydrochloride phenol solution was added to a crude solution in a drum having an adjusted concentration, and bisphenol A with an adjusted basicity corresponding to an amount of 2 x $10^{-7}$

moles of bisphenol A as disodium salt per mole of BPA was continuously manufactured.

In this case, while continuously measuring the basicity of the pelletized bisphenol A obtained in process (h) above, the basicity was adjusted by controlling the amount of the 0.5% sodium hydroxide phenol solution added in process (c) above so that the basicity corresponded to an amount of 2 x $10^{-7}$ moles of bisphenol A as disodium salt per mole of BPA.

Manufacturing of polycarbonate

Using molten bisphenol A continuously fed in from process (g) via a direct line, polymerization of the above polycarbonate was carried out continuously for 14 days to manufacture polycarbonate.

The bisphenol A and the polycarbonate were sampled at 2-hour intervals, and the purity and color tone of the bisphenol A and the color tone and MFR of the polycarbonate were evaluated. The results are shown in Table 1.

Example 2

Bisphenol A was continuously manufactured in the same manner as in Example 1, except that the 0.5% sodium hydroxide phenol solution was added to a slurry (the tube through which the slurry was flowing) containing the addition compound crystals obtained in process (d), and polycarbonate was manufactured in the same manner as in Example 1. The results are shown in Table 1.

Example 3

Bisphenol A was continuously manufactured in the same manner as in Example 1, except that the 0.5% sodium hydroxide phenol solution was added via a tube for removing the addition compound crystals obtained by centrifugation and vacuum filtering in process (e), and polycarbonate was manufactured in the same manner as in Example 1.

Example 4

Bisphenol A was continuously manufactured in the same manner as in Example 1, except that the 0.5% sodium hydroxide phenol solution was added to a dissolving tank for the addition compound crystals in process (f), and polycarbonate was manufactured in the same manner as in Example 1. The results are shown in Table 1.

Comparison Example 1

Bisphenol A was continuously manufactured by the above manufacturing process without adding an alkali(ne earth) metal compound. The color tone and purity of this bisphenol A are shown in Table 1.

Next, the above molten bisphenol A continuously fed in from process (g) via a direct line was introduced into the stirring tank, disodium salt of bisphenol A was added in an amount of 4 x $10^{-7}$ moles with respect to 1 mole of bisphenol A, and polycarbonate was continuously manufactured for 14 days by the above polycarbonate polymerization method. The results are shown in Table 1.

Comparison Example 2

Bisphenol A was manufactured in the same manner as in Example 4, except for the fact that measurement of the basicity of the manufactured bisphenol A and control of the amount of the alkali(ne earth) metal compound added were not carried out, and in process (f), the 0.5% sodium hydroxide phenol solution was added in the specified amount of 4 x $10^{-7}$ moles of disodium salt of bisphenol A per mole of BPA, and polycarbonate was manufactured using this bisphenol A. The results are shown in Table 1.

Comparison Example 3

Bisphenol A was manufactured in the same manner as in Example 4, except that the basicity of the bisphenol A manufactured was adjusted to an amount corresponding to 2 x $10^{-6}$ moles of bisphenol A as disodium salt per mole of BPA, and polycarbonate was manufactured using this bisphenol A. The results are shown in Table 1.

When the amount of the alkali or alkaline earth metal compound used is high, as in Comparison Example 3, the color tone of the bisphenol A and yellowing index (YI) of the polycarbonate are inferior.

Table 1

| | Bisphenol A | | | Polycarbonate | |
|---|---|---|---|---|---|
| | Basicity (Moles of • 2Na/moles of BPA) | Color Tone* ALPHA | Purity* (%) | Color Tone* (Yellowing Index, YI) | MFR* (g/10min) |
| Example 1 | $2 \times 10^{-7}$ mol | 20±5 | 99.92 ± 0.04 | 0.90±0.02 | 11.0 ± 0.4 |
| Example 2 | $2 \times 10^{-7}$ mol | 20±5 | 99.92 ± 0.03 | 0.91±0.02 | 11.0 ± 0.4 |
| Example 3 | $2 \times 10^{-7}$ mol | 20±5 | 99.93 ± 0.04 | 0.91±0.02 | 11.0 ± 0.4 |
| Example 4 | $2 \times 10^{-7}$ mol | 20±5 | 99.92 ± 0.05 | 0.90 ± 0.02 | 11.0 ± 0.4 |
| Comparative Example 1 | (not added) | 25±10 | 99.88 ± 0.07 | 0.91±0.08 | 11.5 ± 1.3 |
| Comparative Example 2 | $4 \times 10^{-7}$ mol (amt added) | 20±10 | 99.88 ± 0.07 | 0.90±0.06 | 11.5 ± 1.1 |
| Comparative Example 3 | $2 \times 10^{-6}$ mol (amt added) | 35±5 | 99.78 ± 0.11 | 1.22±0.04 | 11.0 ± 0.4 |
| *Mean and standard deviations for data collected every 2 h for 14 days. | | | | | |

Example 5

Bisphenol A was manufactured in the same manner as in Example 4, except that the basicity of the manufactured bisphenol A was adjusted to 4 x $10^{-7}$ moles of bisphenol A as disodium salt per mole of BPA, and polycarbonate was manufactured using this bisphenol A. The results are shown in Table 2.

Example 6

Bisphenol A was manufactured in the same manner as in Example 4, except that the basicity of the manufactured bisphenol A was adjusted to 8 x $10^{-7}$ moles of bisphenol A as disodium salt per mole of BPA, and polycarbonate was manufactured using this bisphenol A. The results are shown in Table 2.

Example 7

Bisphenol A was manufactured in the same manner as in Example 4, except that the basicity of the manufactured bisphenol A was adjusted to 8 x $10^{-7}$ moles of bisphenol A as disodium salt per mole of BPA.

In manufacturing polycarbonate using this bisphenol A, in order to compensate for polymerization activity, the 0.5% sodium hydroxide phenol solution was added to the reactor for manufacturing polycarbonate (stirring tank) in an amount of 4 x $10^{-7}$ moles as disodium salt per mole of BPA. The results are shown in Table 2.

Comparison Example 4

Bisphenol A was manufactured by the same method as in Example 4, except that the basicity of the manufactured bisphenol A was adjusted to 2x$10^{-6}$ moles of bisphenol as disodium salt A per mole of BPA, and polycarbonate was manufactured using this bisphenol A. The results are shown in Table 2.

Table 2

| | Bisphenol A | | | Polycarbonate | |
|---|---|---|---|---|---|
| | Basicity (Moles of ··2Na/ moles of BPA) | Color tone* APHA | Purity* (%) | Color Tone* (Yellowing Index, YI) | MFR* (g/10min) |
| Example 5 | $4 \times 10^{-7}$ | 20 ± 5 | 99.92 ±0.03 | 0.91 ± 0.02 | 11.0 ± 0.4 |
| Example 6 | $8 \times 10^{-7}$ | 20 ± 5 | 99.92±0.05 | 0.91 ± 0.02 | 11.0 ± 0.4 |

(continued)

| | Bisphenol A | | | Polycarbonate | |
|---|---|---|---|---|---|
| | Basicity (Moles of ··2Na/ moles of BPA) | Color tone* APHA | Purity* (%) | Color Tone* (Yellowing Index, YI) | MFR* (g/10min) |
| Example 7 | $8 \times 10^{-8}$ | 20 ± 5 | 99.93±0.03 | 0.91 ± 0.02 | 11.0 ± 0.4 |
| Comparison Example 4 | $2 \times 10^{-8}$ | 40 ± 5 | 99.65±0.03 | 1.25 ± 0.02 | 11.0 ± 0.4 |
| *Mean and standard deviations for data collected every 2 h for 14 days. | | | | | |

**Claims**

**1.** A process for manufacturing a bisphenol by reacting a phenol and a ketone comprising:

adding to the bisphenol obtained by reacting the phenol and the ketone, an alkali metal compound or alkaline earth metal compound;
thereby adjusting the basicity to be equivalent to a bisphenol/bisphenol disodium salt mixture comprising an amount of from $1x10^{-8}$ to $1x10^{-6}$ moles of bisphenol disodium salt with respect to 1 mole of bisphenol, wherein the amount of the alkali metal compound or alkaline earth metal compound added is controlled based on measuring the basicity of the bisphenol finally obtained.

**2.** A process for manufacturing a bisphenol according to Claim 1 wherein the basicity of the bisphenol is determined based on measuring the degree of transesterification when the bisphenol and a carbonic diester are subjected to transesterification using only the alkali metal or alkaline earth metal contained in the bisphenol as a transesterification catalyst.

**3.** A process for manufacturing a bisphenol according to Claim 1 comprising:

(a) reacting a phenol and a ketone in the presence of an acidic catalyst to form a reaction mixture comprising bisphenol, catalyst and low boiling substances;
(b) removing from the reaction mixture the catalysts and low-boiling-point substances;
(c) obtaining from the reaction mixture remaining after the catalysts and low-boiling-point substances have been removed a homogeneous solution with an adjusted concentration of bisphenol by adding or removing phenol;
(d) cooling the homogeneous solution obtained and crystallizing an addition compound of bisphenol and phenol to form a slurry;
(e) subjecting the slurry to solid-liquid separation to obtain an addition compound of bisphenol and phenol in the form of a solid;
(f) heating and melting the solid addition compound to form a molten mixture containing phenol; and
(g) removing the phenol from the molten mixture to obtain the bisphenol;

wherein an alkali metal compound or alkaline earth metal compound is added in at least one of (c), (d), (e) or (f).

**4.** A process for manufacturing a bisphenol according to Claim 1, wherein the bisphenol is bisphenol A.

**5.** A process for manufacturing a bisphenol according to Claim 4, wherein the basicity of the bisphenol A is adjusted to within 10% of a specified equivalent basicity value which is equivalent to the basicity of a bisphenol/bisphenol disodium salt mixture comprising from $1 \times 10^{-8}$ to $1 \times 10^{-6}$ moles of bisphenol as disodium salt with respect to 1 mole of the bisphenol.

**6.** A process for manufacturing a bisphenol according to Claim 1, wherein the bisphenol is bisphenol F.

**Patentansprüche**

1. Verfahren zum Herstellen eines Bisphenols durch Umsetzen eines Phenols und eines Ketons, umfassend:

   Hinzugeben einer Alkalimetall-Verbindung oder Erdalkalimetall-Verbindung zu dem durch Umsetzen des Phenols und des Ketons erhaltenen Bisphenol, wodurch die Basizität auf Äquivalenz zu einer Bisphenol/Bisphenoldinatriumsalz-Mischung, umfassend eine Menge von 1 x $10^{-8}$ bis 1 x $10^{-6}$ Molen Bisphenoldinatriumsalz mit Bezug auf 1 Mol Bisphenol, eingestellt wird, wobei die Menge der hinzugegebenen Alkalimetall-Verbindung oder Erdalkalimetall-Verbindung auf der Grundlage der Messung der Basizität des schließlich erhaltenen Bisphenols kontrolliert wird.

2. Verfahren zum Herstellen eines Bisphenols nach Anspruch 1, worin die Basizität des Bisphenols auf der Grundlage der Messung des Grades der Umesterung bestimmt wird, wenn das Bisphenol und ein Carbonsäurediester einer Umesterung unter Einsatz nur des Alkalimetalls oder Erdalkalimetalls, das in dem Bisphenol als ein Umesterungs-Katalysator enthalten ist, unterworfen wird.

3. Verfahren zum Herstellen eines Bisphenols nach Anspruch 1, umfassend:

   (a) Umsetzen eines Phenols und eines Ketons in Gegenwart eines sauren Katalysators zur Bildung einer Bisphenol, Katalysator und niedrig siedende Substanzen umfassenden Reaktionsmischung,
   (b) Entfernen des Katalysators und der niedrig siedenden Substanzen aus der Reaktionsmischung,
   (c) Herstellen einer homogenen Lösung mit einer eingestellten Konzentration von Bisphenol aus der nach dem Entfernen der Katalysatoren und niedrig siedenden Substanzen übrig gebliebe-nen Lösung durch Hinzugeben oder Entfernen von Phenol,
   (d) Abkühlen der erhaltenen homogenen Lösung und Kristallisieren einer Additionsverbindung von Bisphenol und Phenol zur Bildung einer Aufschlämmung,
   (e) Unterwerfen der Aufschlämmung einer Feststoff-Flüssigkeits-Trennung, um eine Additionsverbindung von Bisphenol und Phenol in Form eines Feststoffes zu erhalten,
   (f) Erhitzen und Schmelzen der festen Additionsverbindung zur Bildung einer Phenol enthaltenden geschmolzenen Mischung und
   (g) Entfernen des Phenols aus der geschmolzenen Mischung, um das Bisphenol zu erhalten, wobei eine Alkalimetall-Verbindung oder Erdalkalimetall-Verbindung bei mindestens einer der Stufen (c), (d), (e) oder (f) hinzugegeben wird.

4. Verfahren zum Herstellen eines Bisphenols nach Anspruch 1, worin das Bisphenol Bisphenol A ist.

5. Verfahren zum Herstellen eines Bisphenols nach Anspruch 4, worin die Basizität des Bisphenol A auf innerhalb von 10% eines spezifizierten äquivalenten Basizitätswertes eingestellt wird, der äquivalent ist der Basizität einer Bisphenol/Bisphenoldinatriumsalz-Mischung, umfassend von 1 x $10^{-8}$ bis 1 x $10^{-6}$ Mole Bisphenol als Dinatriumsalz mit Bezug auf 1 Mol des Bisphenols.

6. Verfahren zum Herstellen eines Bisphenols nach Anspruch 1, worin das Bisphenol Bisphenol F ist.

**Revendications**

1. Procédé de préparation d'un bisphénol par réaction d'un phénol avec une cétone, qui comprend le fait d'ajouter, au bisphénol obtenu par réaction du phénol et de la cétone, un dérivé de métal alcalin ou un dérivé de métal alcalino-terreux, pour ajuster ainsi la basicité de façon à ce qu'elle corresponde à celle d'un mélange de bisphénol et de sel disodique de bisphénol contenant 1 x $10^{-8}$ à 1 x $10^{-6}$ mole de sel disodique de bisphénol pour une mole de bisphénol, la quantité de dérivé de métal alcalin ou de dérivé de métal alcalino-terreux à ajouter étant ajustée d'après la mesure de la basicité du bisphénol finalement obtenu.

2. Procédé de préparation d'un bisphénol selon la revendication 1, dans lequel on détermine la basicité du bisphénol en se basant sur la mesure du degré de transestérification lorsque l'on soumet le bisphénol et un diester de l'acide carbonique à une transestérification en utilisant seulement comme catalyseur de transestérification le métal alcalin ou le métal alcalino-terreux contenu dans le bisphénol.

**3.** Procédé de préparation d'un bisphénol selon la revendication 1, qui comprend les étapes consistant à :

a) faire réagir un phénol et une cétone en présence d'un catalyseur acide pour former un mélange réactionnel contenant du bisphénol, le catalyseur et des substances à bas point d'ébullition,
b) éliminer du mélange réactionnel le catalyseur et les substances à bas point d'ébullition,
c) obtenir, à partir du mélange réactionnel restant après l'élimination du catalyseur et des substances à bas point d'ébullition, une solution homogène à concentration ajustée de bisphénol, par addition ou élimination de phénol,
d) refroidir la solution homogène obtenue et faire cristalliser un produit d'addition de bisphénol et de phénol pour former une bouillie,
e) soumettre la bouillie à une séparation solide-liquide pour obtenir un produit d'addition de bisphénol et de phénol sous forme d'un solide,
f) chauffer et faire fondre le produit d'addition solide pour former un mélange fondu contenant du phénol,
g) et éliminer le phénol du mélange fondu pour obtenir le bisphénol, et dans lequel procédé l'on ajoute un dérivé de métal alcalin ou un dérivé de métal alcalino-terreux lors d'au moins l'une des étapes (c), (d), (e) et (f).

**4.** Procédé de préparation d'un bisphénol selon la revendication 1, dans lequel le bisphénol est le bisphénol A.

**5.** Procédé de préparation d'un bisphénol selon la revendication 4, dans lequel on ajuste la basicité du bisphénol A de façon à ce qu'elle diffère de moins de 10 % d'une valeur de basicité spécifiée qui équivaut à la basicité d'un mélange de bisphénol et de sel disodique de bisphénol contenant $1 \times 10^{-8}$ à $1 \times 10^{-6}$ mole de bisphénol sous forme de sel disodique pour une mole de bisphénol.

**6.** Procédé de préparation d'un bisphénol selon la revendication 1, dans lequel le bisphénol est le bisphénol F.

Figure 1

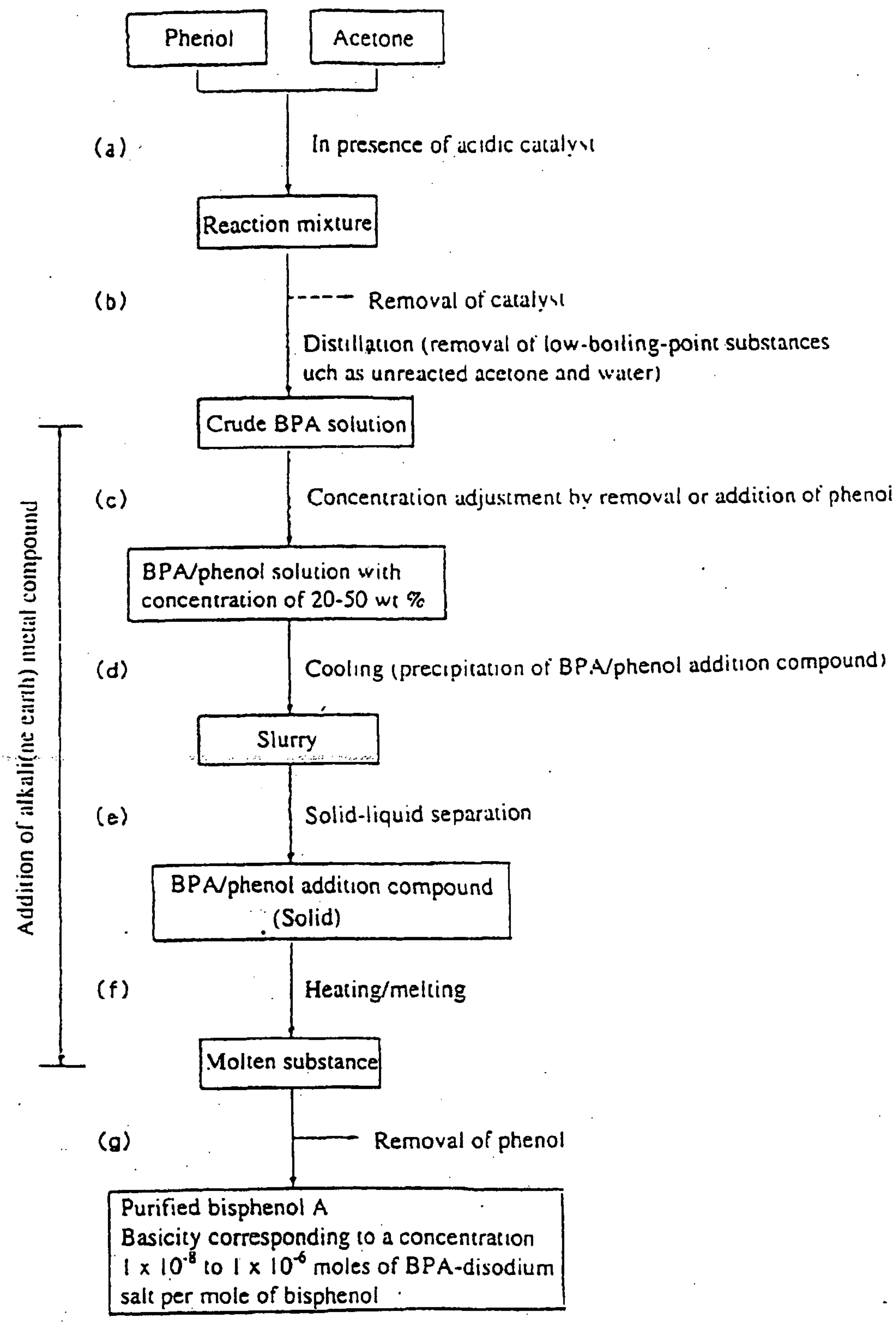
Phenol
Acetone
(a)
In presence of acidic catalyst
Reaction mixture
(b)
Removal of catalyst
Distillation (removal of low-boiling-point substances uch as unreacted acetone and water)
Crude BPA solution
(c)
Concentration adjustment by removal or addition of phenol
BPA/phenol solution with concentration of 20-50 wt %
(d)
Cooling (precipitation of BPA/phenol addition compound)
Slurry
(e)
Solid-liquid separation
BPA/phenol addition compound (Solid)
(f)
Heating/melting
Molten substance
(g)
Removal of phenol
Purified bisphenol A
Basicity corresponding to a concentration 1 x 10-8 to 1 x 10-6 moles of BPA-disodium salt per mole of bisphenol
Addition of alkali(ne earth) metal compound